# EUROPÄISCHE PATENTSCHRIFT

(11) **EP 0 844 232 B1**
(45) Veröffentlichungstag und Bekanntmachung des Hinweises auf die Patenterteilung: **17.01.2001**
(21) Anmeldenummer: 97119989.8
(22) Anmeldetag: 14.11.1997
(51) Int. Cl.: C07C 67/333, C07C 69/716, C07C 69/675

(54) **Verfahren zur Herstellung von Glyoxylsäureestern und deren Hydraten**
Process for the preparation of glyoxylic acid esters and their hydrates
Procédé de préparation d'esters de l'acide glyoxylique et leurs hydrates

(30) Priorität: 21.11.1996 AT 203296
(43) Veröffentlichungstag der Anmeldung: 27.05.1998
(73) Patentinhaber: DSM Fine Chemicals Austria GmbH, 4021 Linz (AT)
(72) Erfinder: Kloimstein, Engelbert, Ing., 4070 Eferding (AT); Giselbrecht Karl Heinz, 4061 Pasching (AT); Hendel, Wolfram, Dr., 4060 Leonding (AT); Perndorfer, Eduard, 4050 Traun (AT); Reiter, Klaus, 4020 Linz (AT); Burger, Christian, 4060 Leonding (AT); Praus, Antonia, 4020 Linz (AT)
(74) Vertreter: Klostermann, Ingrid

(56) Entgegenhaltungen:
- EP-A- 0 293 127
- WO-A-96/22960
- PATENT ABSTRACTS OF JAPAN vol. 17, no. 513 (C-1111), 16.September 1993 & JP 05 140030 A (AGENCY OF IND SCIENCE &TECHNOL.), 8.Juni 1993,

## Beschreibung

Glyoxylsäureester, wie etwa Ethyl-, Methyl- und Benzylglyoxylat oder L-(-)-Menthylglyoxylat sind wichtige Reagentien in der organischen Chemie, da die α-Keto-Ester Gruppe eine sehr reaktive Gruppe darstellt, die an einer Vielzahl von Reaktionen teilnehmen kann. L-(-)-Menthylglyoxylat ist beispielsweise ein wichtiger C₂-Baustein für die asymmetrische Synthese, für chirale Acetale, wie etwa Oxathiolane, für stereokontrollierte Additionsreaktionen an Alkenen und Nitroalkanen oder für Grignard-Reaktionen.

Die Herstellung von Glyoxylsäureestern aus den entsprechenden Maleinsäure- oder Fumarsäurediestern, mittels eines zweistufigen Ozonolyse- und Reduktionsprozesses ist bereits aus mehreren Literaturstellen bekannt.

So werden beispielsweise gemäß J. Org. Chem. 1982, 47, S 891 - 892 Ethyl-, Methyl- oder Benzylglyoxylate durch Ozonolyse der entsprechenden Maleinsäurediester in Dichlormethan, anschließender Reduktion des Ozonides mittels Dimethylsulfid und nachfolgender Destillation erhalten.

Gemäß WO 96/22960 ist ebenfalls ein zweistufiges Verfahren zur Herstellung von Menthylglyoxylat als Zwischenprodukt für Dihydroxyessigsäurementhylester beschrieben, bei welchem Dimenthylmaleat oder -fumarat in der ersten Stufe in einem Halogenkohlenwasserstoff oder Carbonsäureester, bevorzugt in Gegenwart eines niederen aliphatischen Alkohols ozonisiert wird und in der zweiten Stufe das entstandene Ozonolyseprodukt entweder mit einem Dialkylsulfid oder durch katalytische Hydrierung mit Wasserstoff zu Menthylglyoxylat reduziert wird.

Der Nachteil bei den bisher bekannten Verfahren ist jedoch, daß nach dem Ozonolyseschritt peroxidhaltige Ozonolyseprodukte vorliegen, die anschließend in einem zweiten Schritt, entweder mittels katalytischer Hydrierung oder in Anwesenheit von Dialkyl- oder Arylsulfiden, Trialkylphosphiden, zu den entsprechenden Glyoxylsäureestern reduziert werden müssen.

Unerwarteterweise wurde nun gefunden, daß ein wasserlösliches Salz eines Maleinsäure- oder Fumarsäuremonoesters oder von Gemischen derselben, in wäßriger Lösung zu den entsprechenden Glyoxylaten ozonisiert werden kann, wobei die sich bildenden Peroxide rasch zerfallen und der bisher notwendige Reduktionsschritt entfällt.

Gegenstand der vorliegenden Erfindung ist demnach ein Verfahren zur Herstellung von Glyoxylsäureestern oder deren Hydrate, das dadurch gekennzeichnet ist, daß ein wasserlösliches Salz eines Maleinsäure- oder Fumarsäuremonoesters oder eines Gemisches davon in wäßriger Lösung bei Temperaturen von 0 bis 50°C mit Ozon umgesetzt und der entsprechende Glyoxylsäureester oder dessen Hydrat aus dem erhaltenen Reaktionsgemisch isoliert wird.

Als Ausgangsverbindungen werden bei dem erfindungsgemäßen Verfahren Salze von Malein- oder Fumarsäuremonoestern oder Gemische davon verwendet. Geeignete Salze sind solche, die zu wasserlöslichen Verbindungen führen. Beispiele dafür sind Alkali- oder Erdalkalisalze wie etwa Natrium, Kalium-, Kalzium- oder Magnesiumsalze. Bevorzugt werden die Na- oder K-Salze der Maleinsäure- oder Fumarsäuremonoester eingesetzt.

Der Esterteil der erfindungsgemäß eingesetzten Ausgangsverbindungen kann sich dabei sowohl von chiralen als auch von nichtchiralen Alkoholen ableiten. Bei chiralen Alkoholen sind sämtliche zugängliche Stereoisomeren geeignet.

Bevorzugt werden Ester von sekundären oder tertiären Alkoholen, insbesondere von acyclischen, monocyclischen, bicyclischen Terpenalkoholen, von acyclischen, monocyclischen, tricyclischen Sesquiterpenalkoholen, Di- oder Triterpenalkoholen eingesetzt, die gegebenenfalls substituiert sein können.

Besonders bevorzugt sind Ester, die sich von gegebenenfalls verschieden substituierten monocyclischen oder bicyclischen Terpenalkoholen wie etwa von Mentholen, Phenylmenthol, Borneol, Fenchol, u.s.w. ableiten.

Bei dem erfindungsgemäßen Verfahren werden somit bevorzugt Na-, K-, Ca- oder Mg-Salze von Malein- oder Fumarsäuremonoestern eingesetzt, deren Esterteil sich von chiralen oder nicht chiralen, sekundären oder tertiären Alkoholen ableitet.
Dies sind beispielsweise Na-, K-, Ca- oder Mg-Salze des Maleinsäuremonophenylmenthylesters, Maleinsäuremonomenthylesters, Maleinsäuremonofenchylesters, Maleinsäuremonobornylesters u.s.w. bzw. der analogen Fumarsäuremonoester.

Maleinsäure- oder Fumarsäuremonoester sind zum Teil käuflich erhältlich und können als solche direkt in das entsprechende Alkali- oder Erdalkalisalz überführt werden. Die gewünschten Ausgangsverbindungen können aber auch zuerst durch Reaktion von Maleinsäureanhydrid (Fumarsäure) mit dem entsprechenden Alkohol, beispielsweise in der Schmelze bei Temperaturen von 60 bis 120°C oder in einem geeigneten Lösungsmittel, das einen Siedepunkt von ca. 80 bis 120°C aufweist, vorzugsweise in Toluol, hergestellt werden. Die Reaktion kann auch in Gegenwart katalytischer Mengen an Schwefelsäure oder vergleichbarer Säuren durchgeführt werden. Bei der Umsetzung der beiden Reaktanden ist darauf zu achten, daß die Diesterbildung minimiert wird.

Die Herstellung des Maleinsäuremono-L-menthylesters in der Schmelze oder in einem geeigneten Lösungsmittel ist beispielsweise in Annalen d. Chemie, 492, S 273 (1935) oder in Chem. Ber. 119, S 3494 (1986) beschrieben. Die Herstellung weiterer Maleinsäure- und Fumarsäuremonoester kann beispielsweise analog erfolgen.

Die Extraktion der Monoester aus dem Reaktionsgemisch erfolgt mittels Basen, vorzugsweise mittels Natriumhydrogencarbonat oder Natriumhydroxid oder der entsprechenden Base anderer Alkali- oder Erdalkalimetalle.
Die Salze der Malein- und Fumarsäuremonoester mit langen aliphatischen Ketten oder Alicyclen weisen hervorragende Tensideigenschaften auf, sodaß ein Teil des bei der Herstellung der Ester verwendeten Lösungsmittels bei der Extraktion in die wäßrige Phase mitgeführt wird. Da die Anwesenheit dieses Lösungsmittels bei der Ozonolyse jedoch unter Umständen die Kristallisation der entsprechenden Endprodukte, sofern sie kristallin anfallen, erschwert, muß es vor der Ozonolyse durch azeotrope Destillation entfernt werden.
Weiters weisen diese Salze zum Teil eine hohe Neigung zu starkem Schäumen bei Gaseinleitung auf, wodurch der Zusatz eines gegen Ozon beständigen Antischaummittels zur Ozonolyselösung von Vorteil sein kann.
Bevorzugt wird ein Antischaummittel auf Siliconbasis eingesetzt. Die Menge an Antischaummittel beträgt etwa 0,01 - 0,2 Vol%, bevorzugt 0,05 - 0,15 Vol%, bezogen auf die Gesamtmenge an Ozonolyselösung und ist weiters abhängig vom Ausmaß der Neigung zum Schäumen.

Gegebenenfalls können die Salzlösungen vor der Ozonolyse einer weiteren Extraktion bevorzugt mit Toluol unterzogen werden. Die bei der Herstellung der Monoester anfallenden Toluolmutterlaugen die geringe Mengen an Diester und Alkohol enthält, kann wieder in die Veresterung rückgeführt werden.
Weitere geeignete Alkali- oder Erdalkalisalze werden analog erhalten.
Zur Durchführung der Ozonolyse werden die wie oben beschrieben erhaltenen Salzlösungen der Malein- oder Fumarsäuremonoester oder deren Gemischen gegebenenfalls noch mit Wasser verdünnt.

Die Ozonolyse wird bei Temperaturen von 0 bis 50°C durchgeführt, bevorzugt liegt die Temperatur zwischen 10 und 30°C.
Bei dem erfindungsgemäßen Verfahren wird die wäßrige Lösung der jeweiligen Ausgangsverbindungen solange mit einem ozonführenden O₂-Strom begast bis die äquivalente Menge an Ozon bzw. ein leichter Überschuß an Ozon aufgenommen wurde.
Das Ende und somit die Dauer der Reaktion ist durch den Verbrauch der theoretischen Ozonmenge gegeben und läßt sich weiters durch einen gleichzeitig auftretenden erhöhten Ozondurchbruch leicht feststellen.

Bei dem erfindungsgemäßen Verfahren zerfällt das primär entstehende Ozonid bzw. Hydroperoxid sofort in das entsprechende Endprodukt und das entsprechende Spaltprodukt, beispielsweise in das entsprechende Glyoxylsäureesterhydrat und in Natriumoxalat, wobei das Endprodukt frei von Peroxiden ist.

Die Isolierung des entsprechenden Glyoxylsäureesters bzw. dessen Hydrat erfolgt in Abhängigkeit des jeweiligen Aggregatzustandes, in dem das Endprodukt anfällt.

Fällt das entsprechende Endprodukt nach beendeter Ozonolyse als Feststoff an, so wird dieser beispielsweise durch Filtration aus der erhaltenen Suspension isoliert. Im Anschluß daran wird mit Wasser gewaschen und das Endprodukt unter milden Bedingungen, beispielsweise bei Temperaturen von 20 bis 40°C im Vakuum von <10 mbar getrocknet. Je nach Anforderungen an die Reinheit, kann das Endprodukt durch Umkristallisation aus Kohlenwasserstoffen, beispielsweise aus Hexan, oder Ethern, beispielsweise Diisopropylether, weiter gereinigt werden.

Endprodukte, die flüssig anfallen, können beispielsweise mittels Extraktion aus der Ozonolyselösung isoliert werden. Geeignete Extraktionsmittel sind dabei Kohlenwasserstoffe, Ether oder Essigsäureester. Nach erfolgter Extraktion werden die organischen Phasen vereint, mit Wasser gewaschen und getrocknet. Im Anschluß daran wird das Trocknungsmittel abfiltriert, das Lösungsmittel bevorzugt im Vakuum entfernt und das verbleibende Endprodukt getrocknet.

Das erfindungsgemäße Verfahren eignet sich somit zur Ozonisierung von wasserlöslichen Salzen von Maleinsäure- oder Fumarsäuremonoestern oder deren Gemischen. Insbesondere eignet sich das erfindungsgemäße Verfahren zur Herstellung von Glyoxylaten und/oder deren Hydraten chiraler und nichtchiraler Alkohole, bevorzugt sekundärer oder tertiärer Alkohole und besonders bevorzugt von gegebenenfalls verschieden substituierten monocyclischen und bicyclischen Terpenalkoholen.
Die entsprechenden Endprodukte werden dabei in guten Ausbeuten mit hoher Reinheit erhalten.

### Beispiel 1:

### a) Herstellung Maleinsäuremono-L-menthylester

125,02 g (0,8 mol) Maleinsäureanhydrid und 117,67 g L-Menthol wurden in 400 ml Toluol unter Rühren 4 Stunden auf Rückfluß erhitzt.
(GC-Kontrolle, Umsetzung ist beendet, wenn im GC Produktpeak etwa 90 bis 94 Fl.% ausmacht und nicht mehr steigt)
Dann wurde die Reaktionslösung auf Raumtemperatur abgekühlt und zur Entfernung überschüssigen Maleinsäureanhydrids zweimal mit je 200 ml Wasser gewaschen.
Die zweiphasigen Gemische wurden hierbei jeweils etwa 15 Min. bis zur vollständigen Entmischung stehen gelassen.
Anschließend wurde der gebildete Maleinsäuremono-L-menthylester mit 1344 g einer 5 %igen NaHCO₃-Lösung in die wäßrige Phase extrahiert. Die Phasentrennung erforderte etwa 1 Stunde.Geringe Mengen L-Menthol und Diester wurden mit der organischen Phase entfernt, welche in den Prozeß zurückgeführt werden konnten.
Da L-Maleinsäuremonomenthylester aufgrund seiner Tensideigenschaften erhebliche Mengen Toluol in die wäßrige Phase verbrachte, wurden diese durch azeotrope Destillation entfernt.

Gehaltsbestimmung Natrium-monomenthyl maleat:
NMR/HPLC
ca. 1420 - 1460 g Na-Salzlösung, Gehalt (HPLC): 12,5 - 14,0 % enthält ca. 180 - 200 g Natrium monomenthyl maleat (80 - 90 %, Theorie: 221,04 g)

Fp.: 88,4 °C
α_{D}²⁰ = -76,8° (c=1, Acetonitril/Wasser 95:5)
¹H-NMR (CDCl₃): δ = 0,78 (d, J = 7 Hz, 3H, CH₃), 0,88 - 0,95 (br m, 1H, Menthyl-H), 0,91 (d, 3H, CH(CH₃)₂), 0,94 (d, 3H, CH(CH₃)₂), 1,07 - 1,14 (br m, 2H, Menthyl-H), 1,44 - 1,53 (br m, 2H, Menthyl-H), 1,69 - 1,75 (br m, 2H, Menthyl-H), 1,75 - 1,86 (m, 1H, CH(CH₃)₂), 2,04 - 2,08 (br m, 1H, Menthyl-H), 4,86 (dt, J = 4,4 Hz und 10,9 Hz, 1H, COO-CH), 6,35 (d, J = 12,7 Hz, HC = C) 6,45 (d, J = 12,7 Hz, HC = C) ppm

### b) Herstellung Glyoxylsäure-L-menthylester Monohydrat

600 ml (611 g, 0,32 mol) einer wie oben beschrieben hergestellten Natriummonomenthyl-maleat-Lösung wurden mit 400 ml Wasser verdünnt und unter Zusatz von 1ml Entschäumer (Antifoam SRE, Fa. Wacker) bei 33 - 22 °C mit einem Sauerstoffstrom, der 68 g O₃/m³ enthielt, 2 Stunden lang unter Rühren begast. Es wurden 14,8 g Ozon verbraucht (Theorie 14,4 g).
Die aus der Ozonolyse erhaltene weiße Suspension wurde filtriert, der Feststoff mit 100 ml Wasser aufgeschlämmt und trockengesaugt. Der Feststoff wurde noch zweimal mit je 100 ml Wasser gewaschen und bei 30°C im Vakuum < 10 mbar getrocknet.
Es wurden 54,45 g (71 %, bezogen auf eingesetzten L-(-)-Menthol und ozonisierten Anteil) eines weißen Feststoffes erhalten.

Reinheit: 97,1 % (HPLC)

Fp.: 83 - 85 °C (aus Hexan)

α_{D}²⁰ = -74,5° (c = 1, Acetonitril/Wasser 95 : 5)

IR (KBr) = 3423 und 3353 (OH), 2958, 2923, 2872, 2856, 1741 (C = O), 1460, 1376, 1312, 1290, 1224, 1100, 1035 cm⁻¹

### c) Herstellung des Kaliumsalzes

749 g (ca. 0,75 mol, Gehalt 27,8 %) Maleinsäuremono-L-menthylester Natriumsalz-Lösung hergestellt analog 1a) wurden mit 400 ml Wasser verdünnt und mit 153,1 g 30 %iger Schwefelsäure auf pH 1,6 angesäuert. Der verklumpende Niederschlag wurde abgesaugt, zerkleinert und in 1 l Wasser gewaschen und trockengesaugt. 246,2 g feuchter Carbonsäure wurden in 900 ml Wasser aufgenommen und durch Zugabe von 155,4 g 25 %iger Kaliumhydroxid-Lösung in Lösung gebracht. Es wurde auf pH 7,5 eingestellt.

Gehaltsbestimmung Kalium monomenthyl maleat
NMR/HPLC,
1291 g K-Salzlösung Gehalt (HPLC): 15,8 % enthält ca. 204 g Kalium monomenthyl maleat (93 %, Theorie 220,3 g)

### d) Herstellung Glyoxylsäure-L-menthylester Monohydrat

750 ml (0,48 mol) einer Kalium monomenthyl maleat-Lösung hergestellt nach 1c) wurden mit 250 ml Wasser verdünnt und unter Zusatz von 1 ml Entschäumer (Antifoam SRE, Fa. Wacker) bei 20°C mit einem Sauerstoffstrom, der 70 g O₃/m³ enthielt, 2,5 Stunden lang unter Rühren begast. Es wurden 24,4 g Ozon verbraucht (Theorie 23 g).
Die aus der Ozonolyse erhaltene weiße Suspension wurde filtriert, der Feststoff mit 150 ml Wasser aufgeschlämmt und trockengesaugt. Der Feststoff wurde noch zweimal mit je 150 ml Wasser gewaschen und bei 30°C im Vakuum < 10 mbar getrocknet.
Es wurden 35,2 g (32 %, bezogen auf eingesetztes Kaliumsalz) eines weißen Feststoffes erhalten. Durch Extraktion der Mutterlauge (in diesem Fall zweiphasig, da das Produkt nicht vollständig durchkristallisiert war) mit Toluol nach Einstellen des pH auf 2 wurden noch 29,0 g eines farblosen Öls isoliert. Es handelte sich um stark mit Ausgangsmaterial verunreinigtes Produkt, so daß in diesem Fall die Ozonolyse nicht vollständig war.

### Beispiel 2:

### a) Herstellung Maleinsäuremono-D-menthylester

29,42 g (0,3 mol) Maleinsäureanhydrid und 31,25 g (0,2 mol) D(+)-Menthol wurden in 100 ml Toluol unter Rühren 4 Stunden auf Rückfluß erhitzt. (GC-Kontrolle, Umsetzung beendet, als GC Produktpeak 91 Fl.% ausmachte)

Dann wurde die Reaktionslösung auf Raumtemperatur abgekühlt und zur Entfernung überschüssigen Maleinsäureanhydrids zweimal mit je 50 ml Wasser gewaschen.
Die zweiphasigen Gemische wurden hierbei jeweils etwa 15 Min. bis zur vollständigen Entmischung stehen gelassen.
Anschließend wurde der gebildete Maleinsäuremono-D-menthylester mit 336 g einer 5 %igen NaHCO₃-Lösung in die wäßrige Phase extrahiert. Die Phasentrennung erfolgte spontan.

Toluol- und Mentholreste wurden durch azeotrope Destillation entfernt.
328 g = 320 ml Na-Salzlösung
Fp.: 84 - 86 °C (aus Hexan)
α_{D}²⁰ = +79,3° (c = 1, Acetonitril/Wasser 95 : 5)
¹H-NMR (CDCl₃): δ = 0,78 (d, J = 7 Hz, 3H, CH₃), 0,88 - 0,95 (br m, 1H, Menthyl-H), 0,91 (d, 3H, CH(CH₃)₂), 0,94 (d, 3H, CH(CH₃)₂), 1,07 - 1,14 (br m, 2H, Menthyl-H), 1,44 - 1,53 (br m, 2H, Menthyl-H), 1,69 - 1,75 (br, m, 2H, Menthyl-H), 1,75 - 1,86 (m, 1H, CH(CH₃)₂), 2,04 - 2,08 (br m, 1H, Menthyl-H), 4,86 (dt, J = 4,4 Hz und 10,9 Hz, 1H, COO-CH), 6,35 (d, J = 12,7 Hz, HC = C), 6,45 (d, J = 12,7 Hz, HC = C) ppm

### b) Herstellung Glyoxylsäure-D-menthylester Monohydrat

160 ml der Natrium monomenthyl maleat-Lösung wurden mit 850 ml Wasser verdünnt und unter Zusatz von 1 ml Entschäumer (Antifoam SRE, Fa. Wacker) mit einem Sauerstoffstrom, der 29 g O₃/m³ enthielt, bei 20°C 2,25 Std. lang unter Rühren begast. Es wurden 5,4 g Ozon verbraucht (Theorie 4,3 g). Die aus der Ozonolyse erhaltene weiße Suspension wurde filtriert und der Feststoff dreimal mit je 30 ml Wasser gewaschen und bei 30°C im Vakuum < 10 mbar getrocknet.
Es wurden 15,3 g (65 %, bezogen auf eingesetztes D(+)-Menthol und ozonisierten Anteil) eines weißen Feststoffes erhalten.

Reinheit: 97,9 Fl. % (GC), Gehalt: 95,7 % w/w

Fp.: 78,8 °C
α_{D}²⁰ = +74,4° (c = 1, Acetonitril/Wasser 95 : 5)
IR (KBr): 3423 und 3353 (OH), 2959, 2923, 2872, 2856, 1742 (C=O), 1460, 1377, 1234, 1223, 1100, 1035 cm⁻¹

### Beispiel 3:

### a) Herstellung Maleinsäuremonoborneylester

7,35 g (0,075 mol) Maleinsäureanhydrid und 7,71 g (0,05 mol) 1S-endo-(-)-Borneol wurden in 20 ml Toluol unter Rühren 3 Stunden auf Rückfluß erhitzt. (GC-Kontrolle, Umsetzung 93,7 Fl. %, 2,9 Fl. % Borneol)
Dann wurde die Reaktionslösung auf Raumtemperatur abgekühlt und zur Entfernung überschüssigen Maleinsäureanhydrids zweimal mit je 50 ml Wasser gewaschen.
Anschließend wurde der gebildete Maleinsäuremonoborneylester mit 79,8 g einer 5 %igen NaHCO₃-Lösung über 30 Min. in die wäßrige Phase extrahiert. Während der Phasentrennung fiel ein massiver weißer Niederschlag aus, der durch Zugabe von weiteren 300 ml Wasser wieder in Lösung gebracht wurde. Die wäßrige Phase wurde wieder abgetrennt und restliches Toluol durch azeotrope Destillation entfernt.
Es wurden 341,7 g einer wäßrigen Lösung von Natrium monoborneylmaleat erhalten.

141,23 g einer wäßrigen Lösung von Natrium monoborneylmaleat wurden mit 30 ml 1M HCl auf pH 1 gestellt und mit zweimal 100 ml Toluol extrahiert. Die Toluol-Phasen wurden vereinigt und über Na₂SO₄ getrocknet. Nach Abfiltrieren des Trockenmittels und Abziehen des Lösungsmittels im Vakuum wurden 4,8 g (76 %) eines farblosen Öls erhalten, welches beim Stehen über Nacht bei 5°C kristallin erstarrte (Fp.44-48°C).
Umkristallisieren von 4,5 g der Substanz aus 15 ml n-Hexan ergab 3,32 g.
Reinheit 98,4 Fl. % GC
Fp.: 52,3°C
α_{D}²⁰ = -45,1° (c = Acetonitril/Wasser 95 : 5)
¹H-NMR (DMSO): δ = 0,83 (s, 3H, CH₃), 0,87 (s, 3H, CH₃), 0,90 (s, 3H, CH₃), 1,07 (dd, 1H, 3 endo-H), 1,18 - 1,31 (m, 2H, 5 endo-H, 6 endo-H), 1,68 - 1,78 (m, 2H, 4,5 exo-H), 1,85 - 1,94 (m, 1H, 6 exo-H), 2,23 - 2,33 (m, 1H, 3 exo-H), 4,87 (m, 1H, 2 exo-H), 6,33 (d, J= 12,1 Hz, 1H, CH=C), 6,39 (d, J = 12,1 Hz, 1H, CH = C) ppm

### b) Herstellung Glyoxylsäureborneylester Monohydrat

311,2 g der obigen Natriumsalzlösung wurden unter Zusatz von ca. 2 g Antischaummittel (Antifoam SRE, Fa. Wacker) 25 Min. lang bei Raumtemperatur ozonisiert (Ozonverbrauch ca. 7,4 g). Die Lösung wurde nach ca. 10 Min. viskos. Die Ozonolyselösung wurde mit 200 ml Toluol aus der Apparatur gespült und extrahiert. Anschließend wurde nochmals mit 50 ml Toluol extrahiert. Die vereinigten Toluol-Phasen wurden mit 100 ml Wasser gewaschen und über Na₂SO₄ getrocknet. Nach Abfiltrieren des Trocknungsmittels wurde das Lösungsmittel im Vakuum entfernt und der verbleibende ölige Rückstand bei < 1 mbar getrocknet.
Es wurden 5,8 g (61 %) einer öligen Substanz erhalten.
Reinheit (GC): 96,2 Fl.%

IR (KBr): 3450 (OH), 2954, 2881, 1744 (C = O), 1455, 1390, 1221, 1114, 885, 822
¹H-NMR (CDCl₃): δ=0,75, 0,79, 0,81, 0,82, 0,84, 0,85, 0,87 (7s, 9H, 1-CH₃ und C(CH₃)₂), 0,97 - 1,05 (m, 1H, 3 endo-H), 1,15 - 1,30 (m, 2H, 5 endo, 6 endo-H), 1,64 - 1,73 (m, 2H, 4,5 exo-H), 1,83 - 1,90 (m, 1H, 6 exo-H), 2,28 - 2,37 (m, 1H, 3 exo-H), 3,90 - 4,20 (sehr breit, <2H, CH(OH)₂), 4,86 - 5,03 (m, 1H, 2 exo-H), 5,19 - 5,29 (3s, < 1H,-CH(OH)₂), 9,35 (s, ca. 0,2H, -CH = O)

### Beispiel 4:

### a) Herstellung Maleinsäuremonofenchylester

29,42 g (0,3 mol) Maleinsäureanhydrid und 30,85 g (0,2 mol) (1R)-endo-(+)-Fenchylalkohol wurden in 100 ml Toluol unter Rühren 8 Stunden auf Rückfluß erhitzt.
(GC-Kontrolle, Umsetzung 85 Fl.%, 6 % Fenchylalkohol)

Dann wurde die Reaktionslösung auf Raumtemperatur abgekühlt und zur Entfernung überschüssigen Maleinsäureanhydrids zweimal mit je 70 ml Wasser gewaschen. Anschließend wurde der gebildete Maleinsäuremonofenchylester mit 336 g einer 5 %igen NaHCO₃-Lösung über 20 Min. in die wäßrige Phase extrahiert und 15 Min. zur Phasentrennung stehen lassen. Die wäßrige Phase wurde abgetrennt und restliches Toluol durch azeotrope Destillation entfernt. Es wurden 364,2 g einer wäßrigen Lösung von Natrium monofenchylmaleat erhalten.
91 g dieser Lösung wurden mit 12 ml 30 %iger H₂SO₄ auf pH 1,5 gestellt und mit 120 ml Toluol extrahiert. Die Toluol-Phase wurde mit 60 ml Wasser gewaschen und über Na₂SO₄ getrocknet. Nach Abfiltrieren des Trockenmittels, Abziehen des Lösungsmittels im Vakuum sowie Trocknung bei < 1 mbar wurden 11,15 g (88 %) eine klaren, gelblichen, öligen Flüssigkeit erhalten.
Die Substanz kristallisierte im Tiefkühlfach, schmolz jedoch bei Raumtemperatur. Der Fp. lag daher zwischen 0 und 5 °C.
α_{D}²⁰ = + 34,3° (c = 1, Acetonitril/Wasser 95 : 5)
¹H-NMR (DMSO): δ = 0,75 (s, 3H, CH₃), 1,05 (s, 3H, CH₃), 1,08 (s, 3H, CH₃), 1,00 - 1,12 (m, 1H, Fenchyl-H), 1,19 (d, 1H, Fenchyl-H), 1,38 - 1,49 (m, 1H, Fenchyl-H), 1,60 - 1,77 (m, 4H, Fenchyl-H), 4,36 (s, 1H, COO-CH), 6,28 (d, 1H, J = 12,1 Hz, HC = C), 6,46 (d, 1H, J = 12,1 Hz, HC = C) ppm

### b) Herstellung Glyoxylsäurefenchylester Monohydrat

222 g der obigen Natriumsalzlösung wurden mit 128 g Wasser verdünnt und unter Zusatz von ca. 2 g Antischaummittel (Antifoam SRE, Fa. Wacker) 2 Stunden lang bei Raumtemperatur ozonisiert (Ozonverbrauch ca. 6,6 g). Nach ca. 20 Min. wurde die Lösung viskos.
Die Ozonolyselösung wurde mit 300 ml Toluol aus der Apparatur gespült und extrahiert. Die Toluol-Phase wurde mit 100 ml Wasser gewaschen und über Na₂SO₄ getrocknet. Nach Abfiltrieren des Trockenmittels wurde das Lösungsmittel im Vakuum entfernt und der verbleibende ölige Rückstand bei < 1 mbar getrocknet.
Es wurden 1,4 g (48 %) eines gelblichen, leicht getrübten Öls erhalten.
Reinheit GC: 97,1 Fl. %
Das Produkt wurde aus einem Gemisch von 15 ml Diethylether und 100 ml n-Hexan kristallin erhalten. Die Ausbeute betrug hierbei 5,85 g. Durch Einengen der Mutterlauge wurden zwei weitere Kristallfraktionen erhalten, so daß die Ausbeute an kristallinem Material insgesamt 8,59 g betrug.

Fp.: 98°C
α_{D}²⁰ = +32,7° (c = 1, Acetonitril/Wasser 95 : 5)
IR (KBr): 3459 (OH) 2958, 2856, 1734 (C = O), 1463, 1390, 1386, 1237, 1228, 1118, 1023, 992, 977, 632 cm⁻¹

### Beispiel 5:

### a) Herstellung Maleinsäuremono-8-phenylmenthylester

0,59 g (6 mmol) Maleinsäureanhydrid und 0,93 g (4 mmol) (-)-8-Phenylmenthol wurden in 6 ml Toluol unter Rühren 46 Std. auf Rückfluß erhitzt. (GC-Kontrolle, Umsetzung 86 Fl.%, 12, % 8-Phenylmenthol)
Dann wurde die Reaktionslösung auf Raumtemperatur abgekühlt und zur Entfernung überschüssigen Maleinsäureanhydrids zweimal mit je 3 ml Wasser gewaschen.
Anschließend wurde der gebildete Maleinsäuremono-8-phenylmenthylester mit 6,7 g einer 5 %igen NaHCO₃-Lösung über 15 Min. in die wäßrige Phase extrahiert und diese mit 14 ml Wasser verdünnt. Die erhaltene weiße Emulsion wurde zur Phasentrennung über Nacht abgestellt. Die wäßrige Phase wurde abgetrennt und restliches Toluol durch azeotrope Destillation entfernt.
Es wurden 17,65 g einer wäßrigen Lösung von Natrium mono-8-phenylmenthylmaleat erhalten.
3,5 g dieser Lösung wurden mit 0,3 ml 30 %iger H₂SO₄ auf pH 1,5 gestellt und mit 2 ml Toluol extrahiert. Die Toluol-Phase wurde mit 5 ml Wasser gewaschen und über Na₂SO₄ getrocknet. Nach Abfiltrieren des Trocknungsmittels, Abziehen des Lösungsmittels im Vakuum sowie Trocknung bei < 1mbar wurden 0,19 g (68 %) eines klaren, farblosen Öls erhalten.

¹H-NMR (DMSO): δ = 0,85 (d, J = 6,4 Hz, 3H, Phenylmenthyl-CH₃), 0,80 - 1,15 (br m, 4H, Phenylmenthyl-H), 1,20 (s, 3H, C(C₆H₅)CH₃)₂), 1,27 (s, 3H, C(C₆H₅)(CH₃)₂), 1,35 - 1,60 (br m, 4H, Phenylmenthyl-H), 1,89 (br d, 1H, Phenylmenthyl-H), 1,96 - 2,05 (br dt, 1H, Phenylmenthyl-H), 4,76 (dt, J = 4,2 Hz, J = 10,7 Hz, 1H, COO-CH), 5,69 (d, 1H, J = 12,0 Hz, HC = C), 6,21 (d, 1H, J = 12,0 Hz, HC. = C), 7,1 - 7,3 (m, 5H, Ar-H) ppm

### b) Herstellung Glyoxylsäure-8-phenylmenthylester

14,1 g der obigen Natriumsalzlösung wurden unter Zusatz von 2 Tropfen Antischaummittel (Antifoam SRE, Fa. Wacker) 8 Min. lang ozonisiert. Nach ca. 3 Minuten wurde die Lösung viskos. Die Ozonolyselösung wurde mit 20 ml Toluol aus der Apparatur gespült und extrahiert.
Anschließend wurde nochmals mit 10 ml Toluol extrahiert. Die vereinigten Toluol-Phasen wurden zweimal mit je 10 ml Wasser gewaschen und über Na₂SO₄ getrocknet. Nach Abfiltrieren des Trockenmittels wurde das Lösungsmittel im Vakuum entfernt und der verbleibende ölige Rückstand bei < 1 mbar getrocknet.
Es wurden 0,39 g (42 %) eines rosafarbenen Öls erhalten.
Reinheit GC: 96,7 Fl %.

¹H-NMR-Spektren wurden mit einem Bruker 300 MHz-Gerät aufgenommen.

## Patentansprüche

1. Verfahren zur Herstellung von Glyoxylsäureestern oder deren Hydrate, dadurch gekennzeichnet, daß ein wasserlösliches Salz eines Maleinsäure- oder Fumarsäuremonoesters oder eines Gemisches davon in wäßriger Lösung bei Temperaturen von 0 bis 50°C mit Ozon umgesetzt und der entsprechende Glyoxylsäureester oder dessen Hydrat aus dem erhaltenen Reaktionsgemisch isoliert wird.

2. Verfahren nach Anspruch 1, dadurch gekennzeichnet, daß als Salz ein Natrium-, Kalium-, Kalzium- oder Magnesiumsalz eines Maleinsäure- oder Fumarsäuremonoesters oder eines Gemisches davon eingesetzt werden.

3. Verfahren nach Anspruch 1, dadurch gekennzeichnet, daß als Maleinsäure- oder Fumarsäuremonoester Ester von chiralen oder nicht chiralen Alkoholen eingesetzt werden.

4. Verfahren nach Anspruch 3, dadurch gekennzeichnet, daß als Maleinsäure- oder Fumarsäuremonoester Ester von sekundären oder tertiären Alkoholen eingesetzt werden.

5. Verfahren nach Anspruch 3, dadurch gekennzeichnet, daß als Maleinsäure- oder Fumarsäuremonoester Ester von gegebenenfalls verschieden substituierten acyclischen, monocyclischen, bicyclischen Terpen-, Sesquiterpen-, Di- oder Triterpenalkoholen eingesetzt werden.

6. Verfahren nach Anspruch 1, dadurch gekennzeichnet, daß der Ozonolyselösung ein gegen Ozon beständiges Antischaummittel zugesetzt wird.

7. Verfahren nach Anspruch 1, dadurch gekennzeichnet, daß der Glyoxylsäureester oder dessen Hydrat in Abhängigkeit des Aggregatzustandes, in dem der Ester anfällt durch Filtration oder Extraktion mit jeweils anschließender Trocknung aus dem Reaktionsgemisch isoliert wird.

## Claims

1. A process for preparing glyoxylic esters or their hydrates, which comprises reacting a water-soluble salt of a maleic or fumaric monoester or a mixture thereof in aqueous solution at temperatures of from 0 to 50°C with ozone, and isolating the corresponding glyoxylic ester or its hydrate from the resulting reaction mixture.

2. The process as claimed in claim 1, wherein the salt used is a sodium, potassium, calcium or magnesium salt of a maleic or fumaric monoester or a mixture thereof.

3. The process as claimed in claim 1, wherein the maleic or fumaric monoesters used are esters of chiral or nonchiral alcohols.

4. The process as claimed in claim 3, wherein the maleic or fumaric monoesters used are esters of secondary or tertiary alcohols.

5. The process as claimed in claim 3, wherein the maleic or fumaric monoesters used are esters of optionally differently substituted acyclic, monocyclic, bicyclic terpene, sesquiterpene, di- or triterpene alcohols.

6. The process as claimed in claim 1, wherein an ozone-resistant antifoam is added to the ozonolysis solution.

7. The process as claimed in claim 1, wherein the glyoxylic ester or its hydrate is isolated from the reaction mixture by filtration or extraction and subsequent drying in each case, depending on the state of aggregation in which the ester is produced.

## Revendications

1. Procédé de préparation d'esters glyoxyliques ou de leurs hydrates, caractérisé en ce qu'on fait réagir un sel hydrosoluble d'un monoester maléique ou fumarique ou d'un mélange de ceux-ci en solution aqueuse à des températures de 0 à 50°C avec de l'ozone et on isole l'ester glyoxylique correspondant ou son hydrate à partir du mélange de réaction obtenu.

2. Procédé selon la revendication 1, caractérisé en ce qu'on utilise, comme sel, un sel de sodium, de potassium, de calcium ou de magnésium d'un monoester maléique ou fumarique ou un mélange de ceux-ci.

3. Procédé selon la revendication 1, caractérisé en ce qu'on utilise, comme monoester maléique ou fumarique, des esters d'alcools chiraux ou non chiraux.

4. Procédé selon la revendication 3, caractérisé en ce qu'on utilise, comme monoester maléique ou fumarique, des esters d'alcools secondaires ou tertiaires.

5. Procédé selon la revendication 3, caractérisé en ce qu'on utilise, comme monoester maléique ou fumarique, des esters d'alcools terpéniques, sesquiterpéniques, di- ou triterpéniques, acycliques, monocycliques ou bicycliques, éventuellement diversement substitués.

6. Procédé selon la revendication 1, caractérisé en ce qu'on ajoute un agent antimousse résistant à l'ozone à la solution d'ozonolyse.

7. Procédé selon la revendication 1, caractérisé en ce qu'on isole l'ester glyoxylique ou son hydrate à partir du mélange de réaction, d'une manière dépendante de l'état de l'agrégat dans lequel on obtient l'ester, par filtration ou par extraction, suivie respectivement d'un séchage.
